# EUROPEAN PATENT APPLICATION

(11) **EP 4 316 579 A1**
(43) Date of publication of application: **07.02.2024**
(21) Application number: 22780215.4
(22) Date of filing: 17.03.2022
(51) Int. Cl.: A61N 5/067, A61B 1/00, A61B 1/07

(54) **LIGHT TRANSMISSION/RECEPTION PROBE SYSTEM AND LIGHT TRANSMISSION/RECEPTION PROBE**

(30) Priority: 30.03.2021 JP 2021056435
(71) Applicant: FURUKAWA ELECTRIC CO., LTD., Tokyo 100-8322 (JP)
(72) Inventor: IWAMA, Masaki, Tokyo 100-8322 (JP)
(74) Representative: SSM Sandmair
(86) International application number: PCT/JP2022/012467
(87) International publication number: WO 2022/209996

(57) **Abstract**

A light transmission/reception probe system includes, for example: at least a single light source; a light transmission/reception probe including a first end portion in the axial direction, a second end portion on the opposite side of the first end portion in the axial direction, a core, a first cladding that encloses the core, and a leak enabling section that enables leakage of first light, which is output from the light source, which gets coupled with the first cladding in the first end portion, and which is transmitted through the first cladding, from the outer periphery of the first cladding toward the outside in the radial direction; a light receiving unit that receives second light which arrives from the outside, which gets coupled with the core in the second end portion, which is transmitted through the core, and which is output from the core in the first end portion; and a detecting unit that detects the second light received by the light receiving unit.

## Description

### Field

The present invention relates to a light transmission/reception probe system and a light transmission/reception probe.

### Background

Conventionally, a medical probe is known that irradiates a laser light from the front end thereof (for example, refer to Patent Literature 1).

### Citation List

### Patent Literature

[Patent Literature 1] Japanese Patent Application Laid-open No. 2011-104199

### Summary

### Technical Problem

In the probe disclosed in Patent Literature 1, the light radiation position is provided only at the front end of the probe, thereby limiting the light irradiation range only to the vicinity of the front end.

Moreover, using only the probe disclosed in Patent Literature 1, it is not possible to confirm whether the light is being irradiated onto the intended range.

In this type of a probe, it would be beneficial to be able to expand the irradiation range and to be able to confirm whether the light is reaching the intended irradiation range.

In that regard, it is an objective of the present invention to obtain a new and improved light transmission/reception probe system and a new and improved light transmission/reception probe that enable expansion of the irradiation range as well as enable confirmation of the irradiation range.

### Solution to Problem

A light transmission/reception probe system according to the present invention includes, for example: at least one light source; a light transmission/reception probe including a first end portion in an axial direction, a second end portion on an opposite side of the first end portion in the axial direction, a core, a first cladding enclosing the core, and a leak enabling section configured to enable leakage of first light from an outer periphery of the first cladding toward an outside in a radial direction, the first light being output from the light source, coupled with the first cladding in the first end portion, and transmitted through the first cladding; a light receiving unit configured to receive second light, the second light arriving from an outside, being coupled with the core in the second end portion, being transmitted through the core, and being output from the core in the first end portion; and a detecting unit configured to detect the second light received by the light receiving unit.

The light transmission/reception probe system may include an integrated coupling section including a first coupling section facing a position that is deviated from an axis center of the first end portion toward the outside in the radial direction, the first coupling section being configured to couple the light output from the at least one light source with the first cladding, and a second coupling section facing a position closer to the axis center of the first end portion as compared to first coupling section, the second coupling section being configured to couple the light output from the core at the first end portion.

The light transmission/reception probe may include a first enveloping layer configured to enclose the outer periphery of the first cladding in between the first end portion and the leak enabling section.

The first enveloping layer may include a second cladding having a lower refractive index than a refractive index of the first cladding.

The light transmission/reception probe may include an envelope configured to enclose an outermost side of the light transmission/reception probe in the radial direction.

The envelope may be made of a resin material.

The light transmission/reception probe may include a section in which outer periphery of the first cladding is exposed at a position deviated from the leak enabling section in the axial direction.

The light transmission/reception probe may include a second enveloping layer configured to enclose the first cladding on the opposite side of the first end portion in the axial direction with respect to the leak enabling section, and restrict direct coupling of the first light with the core in the leak enabling section.

The light transmission/reception probe system may include an optical element configured to change a direction of the light travelling toward an inside in the radial direction toward the core in the second end portion.

The leak enabling section may include a concave portion or a convex portion on the outer periphery of the first cladding.

The leak enabling section may include a particle or a hole inside the first cladding.

The leak enabling section may include a section in which a shape of the outer periphery goes on changing with advancement in the axial direction.

The leak enabling section may include a bent portion of the first cladding.

The leak enabling section may include a scattering layer arranged on an outside of the first cladding in the radial direction and configured to introduce light coming from the first cladding and cause scattering of the light toward the outside in the radial direction.

The integrated coupling section may be optically connected to the first end portion.

The integrated coupling section may include a fiber bundle formed by bundling: a first transmission optical fiber configured to transmit the first light, the first transmission optical fiber having an end portion serving as the first coupling section; and a second transmission optical fiber configured to transmit the second light, the second transmission optical fiber having an end portion serving as the second coupling section.

The fiber bundle may be a tapered fiber bundle that goes on tapering toward the light transmission/reception probe.

The the at least one light source and the core in the first end portion ,ay be optically connected to each other, and third light output from the light source and transmitted through the core may be outputable from the second end portion.

The light transmission/reception probe system may include: a plurality of light sources serving as the at least one light source; and a control unit configured to switch among light sources for outputting light from among the plurality of light sources.

The plurality of light sources may include a plurality of light sources configured to output the first light.

The at least one light source and the core in the first end portion may be optically connected to each other, third light output from the light source and transmitted through the core may be outputable from the second end portion, and the plurality of light sources may include light sources configured to output the first light and the third light.

The light transmission/reception probe system may include an operation input unit configured to receive an operation input performed by a user, and the control unit may be configured to switch among light sources for outputting light from among the plurality of light sources in response to the operation input performed by the user using the operation input unit.

The light transmission/reception probe may include, as the core, a plurality of cores arranged in parallel.

A light transmission/reception probe according to the present invention includes, for example: a first end portion in an axial direction; a second end portion on an opposite side of the first end portion in the axial direction; a core configured to extend in the axial direction; a first cladding configured to enclose the core and extend in the axial direction; and a leak enabling section disposed closer to the second end portion than the first end portion of the first cladding, and configured to allow leakage of first light from an outer periphery of the first cladding toward an outside in a radial direction, the first light being output from a light source, coupled with the first cladding in the first end portion, and transmitted through the first cladding.

The light transmission/reception probe may include an integrated coupling section including a first coupling section facing a position that is deviated from an axis center of the first end portion toward the outside in the radial direction, the first coupling section being configured to couple the light output from at least one light source with the first cladding, and a second coupling section facing a position closer to the axis center of the first end portion as compared to the first coupling section, and configured to couple the light output from the core at the first end portion.

The light transmission/reception probe may include a first enveloping layer configured to enclose an outer periphery of the first cladding in between the first end portion and the leak enabling section.

The first enveloping layer may include a second cladding having a lower refractive index than a refractive index of the first cladding.

The light transmission/reception probe may include an envelope configured to enclose an outermost side in the radial direction.

The light transmission/reception probe may include a second enveloping layer configured to enclose the first cladding on the opposite side of the first end portion in the axial direction with respect to the leak enabling section, and restrict direct coupling of the first light with the core in the leak enabling section.

The light transmission/reception probe may include, in the second end portion, an optical element configured to change a direction of the light travelling toward an inside in the radial direction toward the core. Advantageous Effects of Invention

According to the present invention, it becomes possible to obtain, for example, a new and improved light transmission/reception probe system and a new and improved light transmission/reception probe that enable expansion of the irradiation range as well as enable confirmation of the irradiation range.

### Brief Description of Drawings

FIG. 1 is an illustrative overall configuration diagram of a light transmission/reception probe system according to an embodiment.
FIG. 2 is an illustrative and schematic cross-sectional view (partial lateral view) of a coupling section and a light transmission/reception probe according to the embodiment.
FIG. 3 is an illustrative explanatory diagram illustrating the irradiation range of the light transmission/reception probe according to the embodiment and illustrating the reception of the return light from an end portion of the irradiation range.
FIG. 4 is an illustrative and schematic cross-sectional view of some part of the coupling section and the light transmission/reception probe according to the embodiment.
FIG. 5 is an illustrative block diagram of the light transmission/reception probe system according to the embodiment.
FIG. 6 is a schematic cross-sectional view of an example of a first cladding of the light transmission/reception probe according to the embodiment.
FIG. 7 is a schematic cross-sectional view of an example of the first cladding of the light transmission/reception probe according to the embodiment.
FIG. 8 is a schematic cross-sectional view of an example of the first cladding of the light transmission/reception probe according to the embodiment.
FIG. 9 is a schematic cross-sectional view of an example of a leak enabling section of the light transmission/reception probe according to the embodiment.
FIG. 10 is a schematic cross-sectional view of an example of the leak enabling section of the light transmission/reception probe according to the embodiment.
FIG. 11 is a schematic cross-sectional view of an example of the front end portion of the light transmission/reception probe according to the embodiment.
FIG. 12 is a schematic cross-sectional view of an example of the front end portion of the light transmission/reception probe according to the embodiment.
FIG. 13 is a schematic cross-sectional view of an example of the front end portion of the light transmission/reception probe according to the embodiment.
FIG. 14 is a schematic cross-sectional view of an example of the front end portion of the light transmission/reception probe according to the embodiment.
FIG. 15 is an overall configuration of some part of a light transmission/reception probe system according to a modification example of the embodiment.
FIG. 16 is an illustrative and schematic cross-sectional view in the leak enabling section of a light transmission/reception probe according to a modification example of the embodiment.

### Description of Embodiments

An exemplary embodiment and modification examples of the present invention are described below. The configurations explained in the embodiment and the modification examples described below as well as the actions and the results (effects) attributed to the configurations are only exemplary. Thus, the present invention can be implemented also using some different configuration than the configurations disclosed in the embodiment and the modification examples described below. Meanwhile, according to the present invention, it becomes possible to achieve at least one of various effects (including secondary effects) that are attributed to the configurations.

The embodiment and the modification examples described below include identical configurations. Hence, due to the identical configurations according to the embodiment and the modification examples, identical actions and identical effects are obtained. Moreover, in the following explanation, the same configurations have the same reference numerals assigned thereto, and their explanation is not given repeatedly.

In the present written description, the ordinal numbers are assigned for distinguishing among the components and the regions, and they do not indicate the priority order or the sequence.

### [Embodiment]

### [Configuration of light transmission/reception probe system]

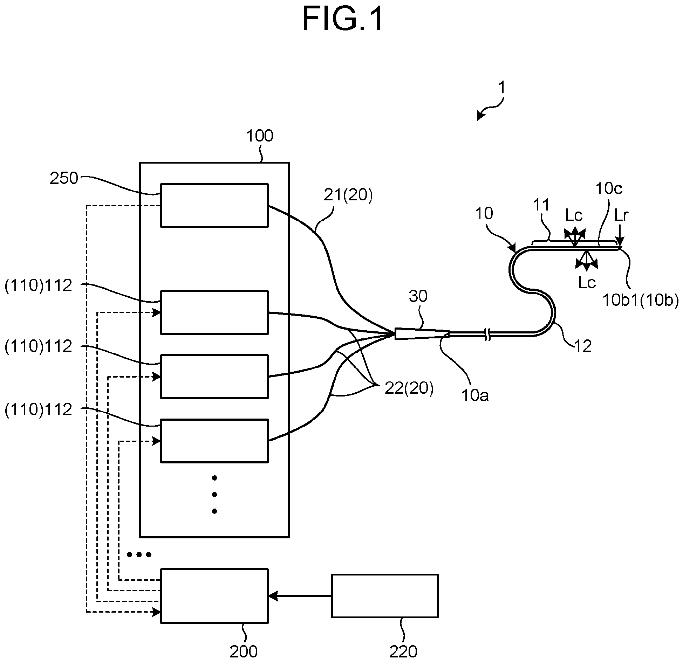

FIG. 1 is a schematic view of a light transmission/reception probe system 1 according to an embodiment. As illustrated in FIG. 1, the light transmission/reception probe system 1 includes a light output device 100, a light transmission/reception probe 10, a control device 200, delivery optical fibers 20, a coupling section 30, an input unit 220, and a light receiving unit 250.

The light output device 100 includes a plurality of light source units 110. Each light source unit 110 includes a light source (not illustrated) for outputting a laser light and includes an optical system (not illustrated) for guiding the light coming from the light source to the corresponding delivery optical fiber 20. The light source includes, for example, a laser element that outputs the laser light. In the present embodiment, as an example, the light output device 100 includes a plurality of light source units 110, that is, a plurality of light sources. However, that is not the only possible case. That is, as long as at least a single light source unit 110 is included, it serves the purpose.

Each light source unit 110 is optically connected to the light transmission/reception probe 10 via the delivery optical fiber 20 disposed corresponding to the light source unit 110 and via the coupling section 30. That is, each delivery optical fiber 20 transmits the light output by the corresponding light source unit 110 to the coupling section 30. In the coupling section 30, the lights that have been transmitted by the delivery optical fibers 20 get coupled with the light transmission/reception probe 10.

The light transmission/reception probe 10 includes an optical fiber; has an elongated, cylindrical, and linear shape; and has flexibility. The light transmission/reception probe 10 has an end portion 10a representing one end in the axial direction, and has an end portion 10b representing the other end in the axial direction. The end portion 10a is adjacent to the coupling section 30 and functions as an input end to which the light coming from the coupling section 30 is input. The end portion 10a can also be referred to as a base end. The end portion 10b is positioned on the opposite side of the end portion 10a in the axial direction, and can be referred to as a front end.

The light transmission/reception probe 10 includes a leak enabling section 11 and a transmitting section 12. The leak enabling section 11 is provided over a predetermined length in the axial direction and at a position distant from the end portion 10a, and is meant for leaking the light from an outer periphery 10c of the light transmission/reception probe 10 toward the outside in radial directions. The transmitting section 12 is provided in between the end portion 10a and the leak enabling section 11 and is meant for transmitting the light.

The control device 200 can control the light source units 110 so as to ensure that, for example, the light source units 110 output the lights or stop outputting the lights. The control device 200 can also control the operations of other devices and parts other than the light source units 110 in the light transmission/reception probe system 1. The input unit 220 constitutes a user interface to be operated by the operator (user); and, according to an operation input performed by the operator, inputs an instruction signal to the control device 200. The input unit 220 represents an example of an operation input unit.

The light receiving unit 250 is optically connected to the end portion 10a, which is on the base end side of a core 10d, via an optical coupler (not illustrated), a delivery optical fiber 21, and the coupling section 30. The light receiving unit 250 is capable of receiving a foreign light that gets coupled with the end portion 10b at the front end side of the core 10d, that gets transmitted through the light transmission/reception probe 10, and that is output from the end portion 10a. The light receiving unit 250 represents, for example, a light receiving element.

### [Configuration of light transmission/reception probe]

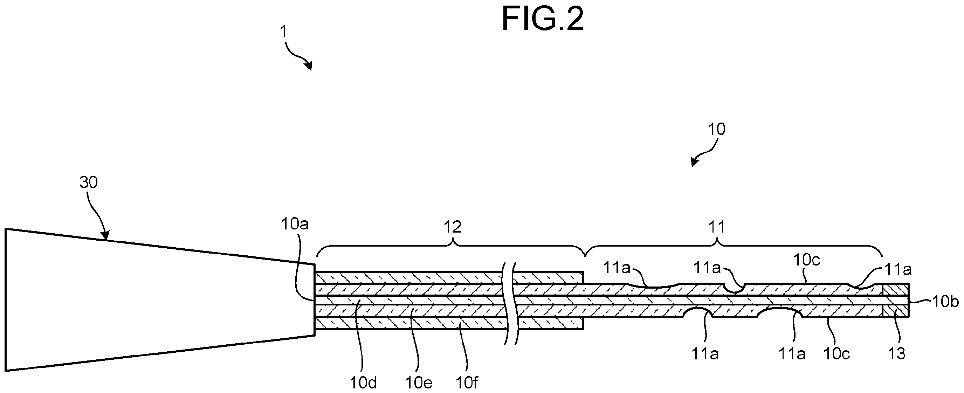

FIG. 2 is a diagram illustrating a cross-sectional surface of the light transmission/reception probe 10 and a lateral surface of the coupling section 30. As explained earlier, the light transmission/reception probe 10 includes the transmitting section 12 and the leak enabling section 11. In the present embodiment, as an example, the transmitting section 12 is configured as a double-clad fiber. That is, the transmitting section 12 includes: the core 10d that extends in the axial direction; a first cladding 10e that encloses the core 10d and extends in the axial direction; and a second cladding 10f that encloses the first cladding and that extends in the axial direction. The refractive index of the first cladding 10e is lower than the refractive index of the core 10d, and the refractive index of the second cladding 10f is lower than the refractive index of the first cladding 10e.

The light input from the coupling section 30 gets coupled with the first cladding 10e at the end portion 10a and, from the transmitting section 12 to the leak enabling section 11, gets transmitted through the first cladding 10e toward the end portion 10b. Moreover, the foreign light arriving from outside gets coupled with the core 10d at the end portion 10b and, from the leak enabling section 11 to the transmitting section 12, gets transmitted through the core 10d toward the end portion 10a. The light transmitted through the first cladding 10e is referred to as first light, and the light transmitted through the core 10d is referred to as second light. The end portion 10a represents an example of a first end portion, and the end portion 10b represents an example of a second end portion.

The second cladding 10f can be, for example, an envelope made of a resin material such as a synthetic resin material having flexibility. Alternatively, the air can serve as the second cladding 10f. In that case, the second cladding 10f is absent in between the end portion 10a and the leak enabling section 11; and, in the transmitting section 12, the outer periphery 10c of the first cladding 10e is exposed at least from the second cladding 10f. Meanwhile, the transmitting section 12 can include an envelope that encloses the second cladding 10f. In these configurations, the second cladding 10f or the envelope represents an example of a first enveloping layer that encloses the outer periphery 10c of the first cladding 10e at least in between the end portion 10a and the leak enabling section 11. Because of the first enveloping layer, it becomes possible to hold down the leakage of the first light from the first cladding 10e. Meanwhile, of the light transmission/reception probe 10, the portion that gets inserted inside a body can be entirely covered by an envelope.

The leak enabling section 11 leaks the first light, which is transmitted through the first cladding 10e, from the outer periphery 10c of the first cladding 10e toward the outside in radial directions. In the present embodiment, the leak enabling section 11 does not include a second cladding. Moreover, in the present embodiment, as an example, in the leak enabling section 11, concave portions 11a are formed on the outer periphery 10c of the first cladding 10e. In that case, the first light gets refracted from the concave portions 11a and changes the travelling direction, that is, becomes scattered; and leak out from the outer periphery 10c toward the outside in radial directions. Meanwhile, on the outer periphery 10c, convex portions can be provided in place of the concave portions 11a. Still alternatively, convex portions can be provided, for example, in between the concave portions 11a. In the present embodiment, the concave portions 11a or the convex portions facilitate leaking of the first light from the first cladding 10e toward the outside in radial directions. Meanwhile, the specifications of the concave portions 11a or the convex portions, such as the installation positions, the installation density, the size, and the depth can be adjusted so as to adjust the distribution of the leakage intensity of the first light in the axial direction of the leak enabling section 11.

The second light that is input from the end portion 10b gets transmitted through the core 10d and is output from the end portion 10a. The first cladding 10e enables holding down the leakage of the second light from the outer periphery of the core 10d.

In this way, the light transmission/reception probe 10 can output first light Lc (see FIG. 1) toward the outside in radial directions via the leak enabling section 11. The leak enabling section 11 can be set to be relatively longer in the axial direction of the light transmission/reception probe 10. Hence, according to the present embodiment, the light irradiation range of the light transmission/reception probe 10 can be expanded more as compared to a configuration in which the light irradiation range is limited to the vicinity of the end portions in the axial direction.

Moreover, the light transmission/reception probe 10 can transmit second light Lr (see FIG. 1) that is input to the end portion 10b of the core 10d. In this way, with a relatively simpler configuration based on optical fibers, the light transmission/reception probe 10 can cater to the transmission of light as well as to the reception of light.

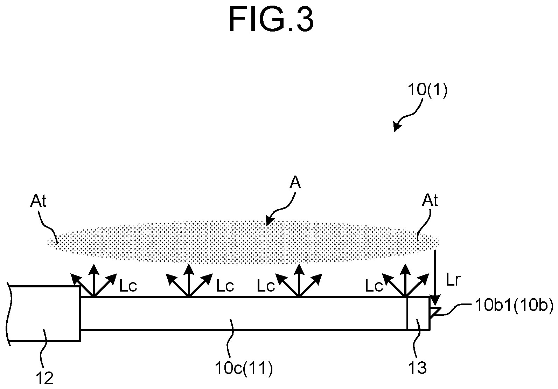

FIG. 3 is a schematic diagram illustrating an irradiation region A of the first light Lc. As illustrated in FIG. 3, the irradiation region A faces the outer periphery 10c in the leak enabling section 11. Meanwhile, in FIG. 3, it is illustrated that the irradiation region A is present only on one side of the outer periphery 10c (in FIG. 3, the upper side). However, in practice, a cylindrical region enclosing the outer periphery 10c can be treated as the irradiation range A. As illustrated in FIG. 3, the end portion 10b is positioned close to an end portion At on the front side of the irradiation range A, and faces the end portion At. Consequently, the second light Lr coming from the end portion At of the irradiation range A can be received in the end portion 10b.

As illustrated in FIGS. 2 and 3, in the end portion 10b of the core 10d, an inclined surface 10b1 is formed that is inclined with respect to an axial direction X. In that case, the second light travelling toward the outside in radial directions reflects from the inclined surface 10b1 in the axial direction. The direction of reflection can be adjusted according to the inclination of the inclined surface 10b1. The inclined surface 10b1 represents an example of an optical element.

With such a configuration, in the light transmission/reception probe system 1 according to the present embodiment, for example, the light receiving unit 250 receives and detects the second light Lr, thereby making it possible to detect and determine the presence or absence of irradiation of the first light Lc onto the irradiation range A and to detect and determine the irradiation intensity. Meanwhile, the second light Lr can be the reflected light of the first Lc from the irradiation range A, or can be the fluorescence caused by the first light Lc in the irradiation region A.

Moreover, as illustrated in FIGS. 2 and 3, in the present embodiment, in the light transmission/reception probe 10, in between the first cladding 10e of the leak enabling section 11 and the end portion 10b of the core 10d, an enveloping layer 13 is provided for enclosing the core 10d. The enveloping layer 13 encloses the core 10d on the opposite side of the end portion 10a in the axial direction with respect to the leak enabling section 11, and enables holding down direct coupling of the first light with the core 10d. In that case, the enveloping layer 13 can include an absorption layer for absorbing light. The absorption layer can be configured as a blackened processing layer made of a copper oxide film. Because of the enveloping layer 13, it becomes possible to prevent a situation in which the first light Lc gets coupled with the core 10d, gets released from the end portion 10b, and is irradiated onto an unintended range. Moreover, because of the enveloping layer 13, it becomes possible to hold down the interference of the first light, which is input to the core 10d, with the second light and to hold down the weakening of the second light. Meanwhile, the enveloping layer 13 can be configured to enclose the outer periphery of the first cladding 10e. The enveloping layer 13 represents an example of a second enveloping layer.

### [Configuration of coupling section]

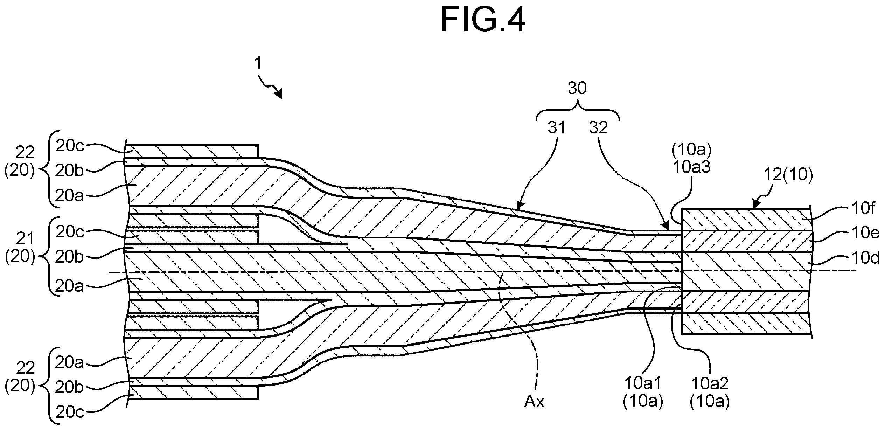

FIG. 4 is a partial cross-sectional view of the coupling section 30 and the light transmission/reception probe 10. The coupling section 30 is connected to the light transmission/reception probe 10 by means of fusion or adhesion. That is, the coupling section 30 is optically and mechanically connected to the light transmission/reception probe 10. Meanwhile, the facets of the coupling section 30 and the facets of the light transmission/reception probe can be configured to be in contact using some other member (not illustrated).

As illustrated in FIG. 4, the coupling section 30 includes a fiber bundle in which a plurality of delivery optical fibers 20 is bundled. The fiber bundle is, for example, a tapered fiber bundle that goes on tapering toward the light transmission/reception probe 10. In FIG. 4, for example, seven delivery optical fibers 20 having the same diameter are bundled according to the closest packing. Each delivery optical fiber 20 includes a core 20a; a cladding 20b that encloses the core 20a; and an envelope 20c that encloses the cladding 20b. The portion in which the envelope 20c is removed constitutes the coupling section 30 that includes a tapered section 31 and a linear section 32. In the tapered section 31, the outer diameter goes on gradually decreasing toward the light transmission/reception probe 10, and the claddings 20b of the adjacent delivery optical fibers 20 are integrated. Meanwhile, the coupling section 30 need not include the linear section 32. Moreover, the number of bundled optical fibers is not limited to seven. That is, as long as the fiber bundle includes the end portions of at least two bundled delivery optical fibers 20, it serves the purpose. Moreover, the fiber bundle can also include other optical fibers, other than the delivery optical fibers 20, that are optically connected to the delivery optical fibers 20.

As illustrated in FIG. 4, of the first cladding 10e of the light transmission/reception probe 10, a facet 10a2 faces a facet of that delivery optical fiber 22 which has deviated from an optical axis Ax toward the outside in a radial direction. The facet 10a2 is a part of the end portion 10a. As illustrated in FIG. 1, the delivery optical fibers 22 are optically connected to light source units 112 (110). The coupling section 30 couples, in the first cladding 10e, the lights output from the light source units 112 and transmitted by the delivery optical fibers 22. That is, the light source of each light source unit 112 outputs the light that gets coupled with the first cladding 10e, that is, outputs the first light. The delivery optical fibers 22 represent examples of a first transmission optical fiber. Of the coupling section 30, the portion in which each delivery optical fiber 22 and the first cladding 10e are optically connected, that is, the end portion of each delivery optical fiber 22 represents an example of a first coupling section.

As illustrated in FIG. 4, in the present embodiment, as an example, a plurality of delivery optical fibers 22 is optically connected to the first cladding 10e. Hence, when a plurality of light source units 112 concurrently outputs lights, the first lights output from the light source units 112 get coupled with the first cladding 10e via the coupling section 30.

As illustrated in FIG. 4, of the core 10d of the light transmission/reception probe 10, a facet 10a1 faces a facet of that delivery optical fiber 22 which is positioned in the middle of the bundle. The facet 10a1 is a part of the end portion 10a. As illustrated in FIG. 1, the delivery optical fiber 21 is optically connected to the light receiving unit 250. Moreover, the coupling section 30 optically connects the core 10d and the delivery optical fiber 21 to each other. Herein, the delivery optical fiber 21 represents an example of a second transmission optical fiber. Of the coupling section 30, the portion in which the core 10d and the delivery optical fiber 21 are optically connected, that is, the end portion of the delivery optical fiber 21 represents an example of a second coupling section. Thus, the coupling section 30 represents an example of an integrated coupling section that includes the first coupling section and the second coupling section.

As illustrated in FIG. 4, a facet 10a3 of the second cladding 10f does not have any facets of the delivery optical fibers 20 facing thereto. On the facet 10a3, a shielding member can be disposed so as to ensure that no foreign light gets coupled with the second cladding 10f.

In the optically-connected portion between each light source unit 112 and the corresponding delivery optical fiber 22, the numerical aperture of the light source unit 112 is set to be substantially same as or slightly smaller than the numerical aperture of the delivery optical fiber 22. Moreover, in the optically-connected portion between the delivery optical fibers 22 (the coupling section 30) and the end portion 10a of the light transmission/reception probe 10, the numerical aperture of the delivery optical fibers 22 is set to be substantially same as or slightly smaller than the numerical aperture of the first cladding 10e. In an identical manner, the numerical aperture of the light receiving unit 250 is set to be substantially same as or slightly smaller than the numerical aperture of the delivery optical fiber 21. Furthermore, in the optically-connected portion between the delivery optical fiber 21 (the coupling section 30) and the end portion 10a of the light transmission/reception probe 10, the numerical aperture of the delivery optical fiber 21 is set to be substantially same as or slightly smaller than the numerical aperture of the core 10d. As a result of setting the numerical apertures in such a way, a reduction is achieved in the loss occurring in the first light and the second light in the optically-connected portions. Herein, the numerical aperture of the first cladding 10e is greater than the numerical aperture of the core 10d. Accordingly, the numerical aperture of the light source units 112 is greater than the numerical aperture of the light receiving unit 250. Meanwhile, the setting of the numerical apertures of the light source units 112 and the light receiving unit 250 can be varied according to, for example, the design regarding the numerical aperture of the lens system (not illustrated).

As explained above, the control device 200 can switch between outputting the light and stopping the output of the light from each light source unit 110. As a result of the control performed by the control device 200 with respect to the operations of each light source unit 110, it becomes possible to switch between the state in which the first light output by the light source units 112 is output from the outer periphery 10c within the leak enabling section 11 of the light transmission/reception probe 10 and the state in which the first light is not output from the light transmission/reception probe 10. Moreover, the control device 200 can vary the number of light source units 112 that output the first light, and accordingly vary the intensity of the first light that leaks out from the outer periphery 10c in the leak enabling section 11.

Regarding changing the operation state and the intensity as explained above, the control device 200 can make the switching based on an instruction signal that is based on an operation input performed by the operator using the input unit 220.

### [Control of irradiation system]

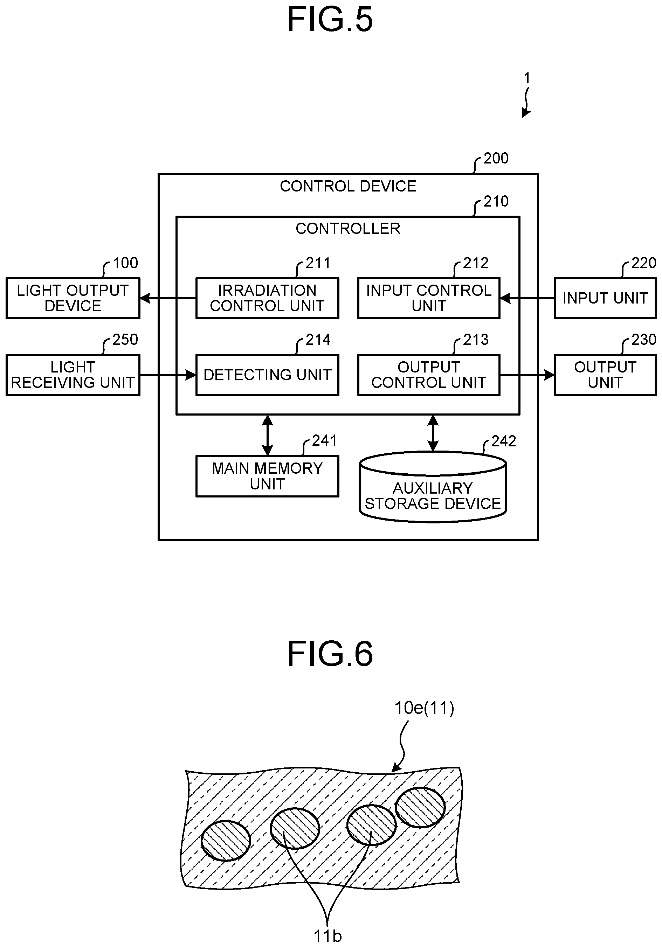

FIG. 5 is a block diagram of the light transmission/reception probe system 1. As illustrated in FIG. 5, the light transmission/reception probe system 1 includes the control device 200, the input unit 220, an output unit 230, and the light receiving unit 250. The input unit 220 and the output unit 230 are used in building a user interface for the user or the operator. The input unit 220 is an input device such as a remote controller; a switchbox; an operating unit such as a joystick; a keyboard; a touch-sensitive panel; a mouse; switches; or operation buttons. The output unit 230 is an output device such as a display, a printer, a lamp, or a speaker for performing output in the form of images, prints, or sounds.

The control device 200 further includes a controller 210, a main memory unit 241, and an auxiliary storage device 242.

The controller 210 is a processor (circuit) such as a CPU (central processing unit). The main memory unit 241 is, for example, a RAM (random access memory) or a ROM (read only memory). The auxiliary storage device 242 is a nonvolatile rewritable storage device such as an SSD (solid state drive) or an HDD (hard disk drive).

The controller 210 reads programs stored in the main memory unit 241 or the auxiliary storage device 242 and executes them, so consequently functions as an irradiation control unit 211, an input control unit 212, an output control unit 213, and a detecting unit 214. The programs can be recorded as installable files or executable files in a computer-readable recording medium. The recording medium can also be called a program product. The values used in the programs or in the arithmetic processing performed by the processors and the information such as maps and tables either can be stored in advance in the main memory unit 241 or the auxiliary storage device 242; or can be stored in a memory unit of a computer connected to a communication network; or can be downloaded via the communication network and stored in the auxiliary storage device 242. The auxiliary storage device 242 is used to store the data written by the processor. Meanwhile, the arithmetic processing performed by the controller 210 can at least be partially performed using hardware. In that case, the controller 210 can include, for example, an FPGA (field programmable gate array) or an ASIC (application specific integrated circuit).

The irradiation control unit 211 can control outputting the light and stopping the output of the light on an individual basis for each light source unit 110 included in the light output device 100. Moreover, according to an operation input performed by the operator using the input unit 220, the irradiation control unit 211 can switch among the light source units 110 for outputting the light from among a plurality of light source units 110 (light sources). The irradiation control unit 211 represents an example of a control unit.

The input control unit 212 receives an input signal from the input unit 220. Moreover, the input control unit 212 can control the input unit 220 so as to enable a predetermined operation input.

The output control unit 213 controls the output unit 230 so as to ensure a predetermined output.

Based on the reception of the second light by the light receiving unit 250, the detecting unit 214 can detect the received-light intensity of the second light.

### [Exemplary configurations of leak enabling section (modification examples)]

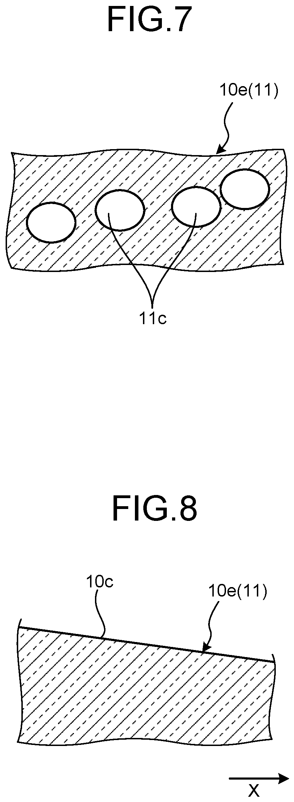

FIGS. 6 and 7 are cross-sectional views illustrating exemplary configurations of the leak enabling section 11. In the example illustrated in FIG. 6, particles 11b are included inside the first cladding 10e of the leak enabling section 11. In the example illustrated in FIG. 7, holes 11c are included inside the first cladding 10e of the leak enabling section 11. The particles 11b and the holes 11c can have, for example, a nanostructure with the diameter equal to or smaller than 100 [nm]. The particles 11b can be, for example, fine particles or fillers such as fine tubes. In these cases, due to the particles 11b or the holes 11c, there occurs a change in the travelling direction of the first light, that is, the first light gets scattered. As a result, it becomes easier for the first light to leak out from the outer periphery 10c toward the outside in radial directions.

FIG. 8 is a cross-sectional view illustrating another exemplary configuration of the leak enabling section 11. In the example illustrated in FIG. 8, the outer periphery 10c is inclined with respect to the axial direction X of the light transmission/reception probe 10. For example, the outer periphery 10c is a tapered surface. In this way, in the portion in which the shape of the outer periphery 10c goes on changing with advancement in the axial direction X; for example, the first light falls at an angle exceeding the critical angle, thereby making it easier for the first light to leak out from the outer periphery 10c toward the outside in radial directions.

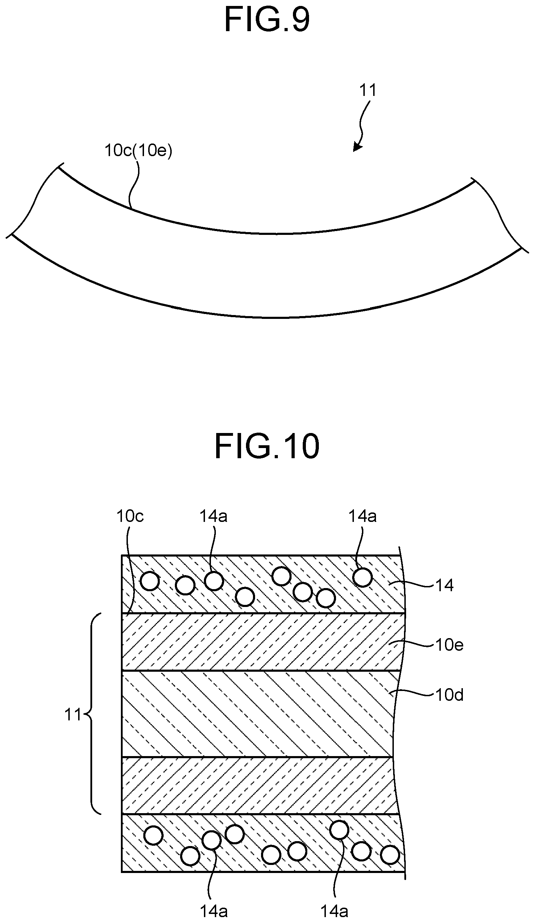

FIG. 9 is a cross-sectional view illustrating still another exemplary configuration of the leak enabling section 11. In the example illustrated in FIG. 9, the leak enabling section 11 is bent. Since light easily leaks out from a bent portion; in the configuration illustrated in FIG. 9 too, it is easier for the first light to leak out from the outer periphery 10c toward the outside in radial directions.

FIG. 10 is a cross-sectional view illustrating still another exemplary configuration of the leak enabling section 11. In the example illustrated in FIG. 10, the leak enabling section 11 includes a scattering layer 14 that encloses the first cladding 10e. The refractive index of the scattering layer 14 is set to be substantially same as or slightly higher than the refractive index of the first cladding 10e. Moreover, in the scattering layer 14, particles or holes are included as scattering elements 14a. In that case, the first light that arrives at the interface between the first cladding 10e and the scattering layer 14 enters the scattering layer 14 and leaks out from the scattering elements 14a toward the outside in radial directions. With such a configuration, for example, the advantage is that the scattering layer 14 can be used to set or vary the spots for leakage of the first light, or the spots for easy leakage of the first light, or the spots at which the intensity of the leaked light is high. Moreover, when the first cladding 10e is moderately pressurized by the scattering layer 14 toward the inside in radial directions, it becomes easier for the first light to leak out from the pressurized portion.

Meanwhile, the configurations illustrated in FIGS. 6 to 10 can also be implemented in combination in a single light transmission/reception probe 10.

### [Exemplary configurations of front end of core of light transmission/reception probe (modification examples)]

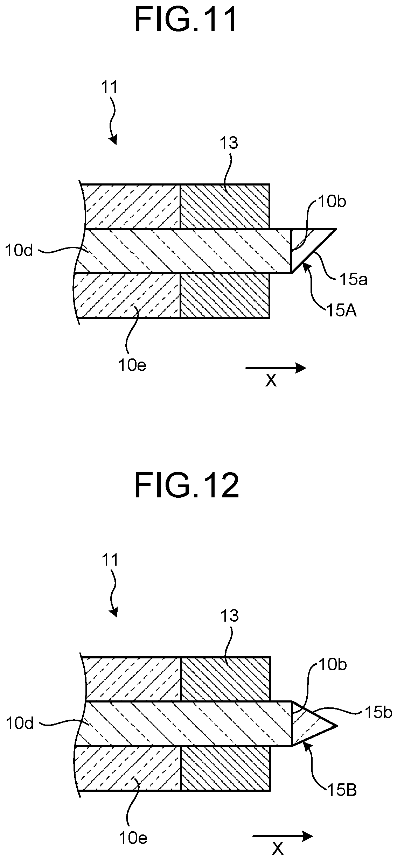

In FIG. 11 is illustrated a configuration in which an optical element 15A having an inclined surface 15a, which is identical to the inclined surface 10b1 illustrated in FIG. 3, is attached to the end portion 10b of the core 10d by means of fusion or adhesion. In that case, the refractive index of the optical element 15A is set to be substantially same as or higher than the refractive index of the core 10d. With such a configuration too, it is possible to achieve the same effects as achieved using the configuration illustrated in FIGS. 2 and 3. Moreover, in the configuration illustrated in FIG. 11, the advantage is that the optical element 15A used in illuminating an appropriate region can be attached at a later timing to the core 10d of the light transmission/reception probe 10. Herein, the inclined surface 15a represents an example of an optical element.

In FIG. 12 is illustrated a configuration in which an optical element 15B having a conical surface 15b, which is protruded in the axial direction X, is attached to the end portion 10b of the core 10d by means of fusion or adhesion. In that case, the refractive index of the optical element 15A is set to be substantially same as or higher than the refractive index of the core 10d. With such a configuration too, it is possible to achieve the same effects as achieved using the configuration illustrated in FIGS. 2 and 3. Moreover, in the configuration illustrated in FIG. 12, the advantage is that the second light can be irradiated onto a wider range surrounding the end portion 10a, such as onto the whole circumference of the end portion 10a. Herein, the conical surface 15b represents an example of an optical element.

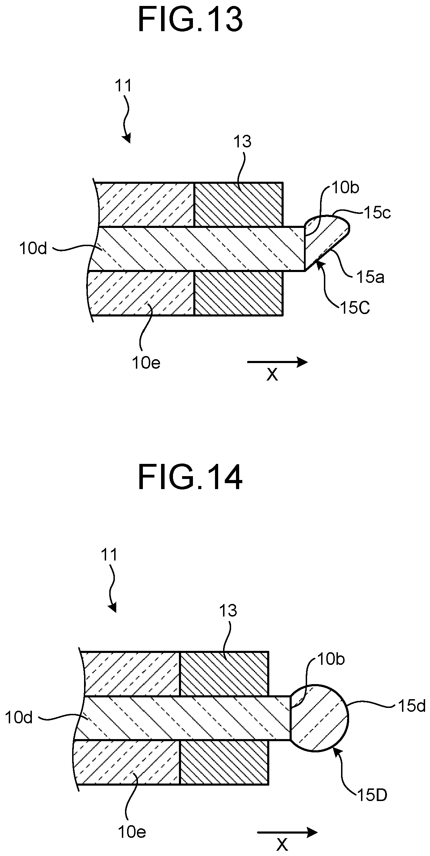

In FIG. 13 is illustrated a configuration in which an optical element 15C, which has the inclined surface 15a identical to FIG. 11 and which includes a lens 15c that refracts the light reflected from the inclined surface 15a, is attached to the end portion 10b of the core 10d by means of fusion or adhesion. In that case, the advantage is that, according to the specifications set for the lens 15c, for example, the irradiation state of the second light can be adjusted, such as the irradiation range of the second light can be narrowed or widened, or the irradiation position can be adjusted, or the irradiation intensity can be increased or reduced. Herein, the lens 15c represents an example of an optical element.

In FIG. 14 is illustrated a configuration in which an optical element 15D having a convex curve 15d, which is like a spherical surface, is attached to the end portion 10b of the core 10d by means of fusion or adhesion. In that case, in an identical manner to the lens 15c illustrated in FIG. 13, the advantage is that, according to the shape of the convex curve 15d, the irradiation state of the second light can be adjusted. Herein, the convex curve 15d represents an example of an optical element.

### [Irradiation of light from front end of light transmission/reception probe (modification example)]

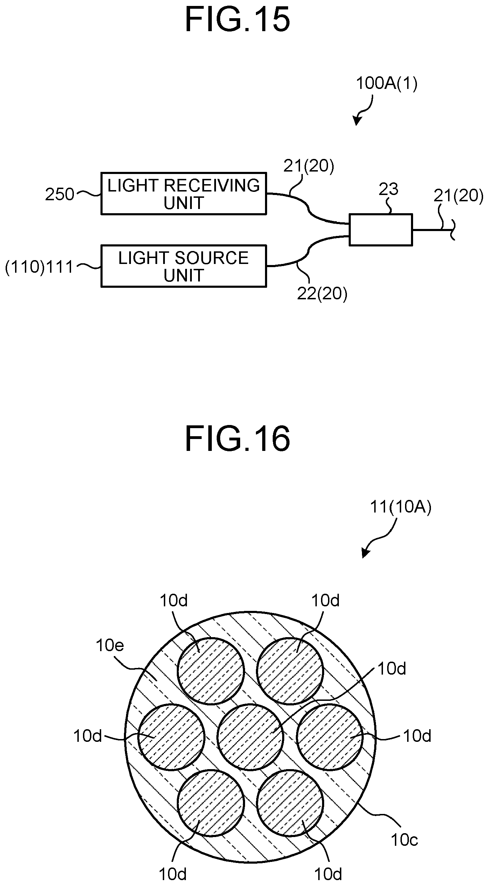

In FIG. 15 is illustrated some part of light output device 100A according to a modification example. In the example illustrated in FIG. 15, a coupler 23 is disposed midway through the delivery optical fiber 21; and the light source unit 111 (110) is optically connected to the core 10d via the delivery optical fiber 22, the coupler 23, and the delivery optical fiber 21. In that case, the light that is output from the light source unit 111 and transmitted through the core 10d (i.e., third light) can be output from the end portion 10b.

In that case, the inclined surface 10b1, or the inclined surface 15a, or the conical surface 15b, or the lens 15c, or the convex curve 15d, or any of the optical elements 15A to 15D that is disposed at the end portion 10b guides the third light, which is travelling in the axial direction, toward the outside in radial directions.

Moreover, in that case, the irradiation control unit 211 can control outputting the light and stopping the output of the light on an individual basis for each light source unit 110 (111, 112) included in the light output device 100. Furthermore, according to an operation input performed by the operator using the input unit 220, the irradiation control unit 211 can switch among the light source units 110 for outputting the light from among a plurality of light source units 110 (111, 112).

### [Modification example of light transmission/reception probe]

FIG. 16 is a cross-sectional view of the leak enabling section 11 of a light transmission/reception probe 10A according to a modification example. As illustrated in FIG. 16, the light transmission/reception probe 10A is configured as a multicore optical fiber that includes a plurality of cores 10d arranged in parallel and includes the first cladding 10e that encloses the cores 10d. In that case, for example, not only the number of cores 10d can be increased, but also the cores 10d can be disposed closer to the irradiation range A. As a result, it becomes possible to increase the received-light intensity of the second light in the light receiving unit 250, and in turn to enhance the detection sensitivity of the second light in the detecting unit 214. Meanwhile, the number of cores 10d is not limited to seven, and can be equal to or greater than two.

As explained above, the light transmission/reception probe system 1 according to the present embodiment enables irradiation of light onto a wider irradiation range A that faces the outer periphery 10c of the light transmission/reception probe 10. That is, according to the present embodiment, the irradiation range A can be expanded to a wider range.

Moreover, the light transmission/reception probe system 1 according to the present embodiment can detect the irradiation intensity of the end portion At of the irradiation range A that faces the end portion 10b of the light transmission/reception probe 10. For example, the light transmission/reception probe system 1 can compare the intensity of the second light Lr, which is coming from the end portion At of the irradiation range A, with a threshold value; and, for example, can determine whether or not the intended largeness of the irradiation range A is achieved or whether or not the intended irradiation intensity is achieved with respect to the irradiation range A. The irradiation intensity with respect to the end portion At is close to the lower limit value of the irradiation intensity with respect to the irradiation range A. Hence, the configuration according to the present embodiment is particularly effective when the irradiation intensity equal to or greater than a predetermined intensity needs to be achieved.

The light transmission/reception probe 10 as well as the light transmission/reception probe system 1 including the light transmission/reception probe 10 can be used in photodynamic therapy (PDT). If the light transmission/reception probe 10 or the light transmission/reception probe system 1 including the light transmission/reception probe 10 is used in PDT; then, for example, it becomes possible to build a valuable medical system that produces various effects such as enabling more reliable treatment or more appropriate treatment.

Moreover, the light transmission/reception probe system 1 according to the present embodiment can also be used in photodynamic diagnosis (PDD). In that case, since the light transmission/reception probe system 1 can be used in PDT as well as PDD; for example, it becomes possible to build a valuable medical system that produces various effects such as compactness, user-friendliness, and enabling appropriate treatment with ease.

More particularly, for example, in the case in which, with respect to a blue light that has the wavelength of 400 [nm] to 410 [nm] and that is irradiated as the first light, a fluorescent-labeled glioma emits red fluorescence having the wavelength of about 600 [nm] as the second light Lr; if the irradiation of the first light Lc and the reception of the second light Lr is performed while pushing in or pulling out the light transmission/reception probe 10, then the existence range of the glioma can be detected as the insertion range for the light transmission/reception probe 10 that has received the second light Lr. As a result, for example, with respect to that existence range, PDT can be performed in a more reliable manner or without waste. In that case, for example, at the time of inserting the light transmission/reception probe 10, the existence range can be detected, that is, PDD can be performed; and, at the time of pulling out the light transmission/reception probe 10, radiation therapy can be performed with respect to the detected existence range, that is, PDT can be performed.

While a certain embodiment has been described, that embodiment and modification examples have been presented by way of example only, and are not intended to limit the scope of the inventions. Indeed, the novel embodiment described herein may be embodied in a variety of other forms; furthermore, various omissions, substitutions and changes in the form of the embodiment described herein may be made without departing from the spirit of the inventions. The accompanying claims and their equivalents are intended to cover such forms or modifications as would fall within the scope and spirit of the inventions. Moreover, regarding the constituent elements, the specifications about the configurations and the shapes (structure, type, direction, shape, size, length, width, thickness, height, number, arrangement, position, material, etc.) can be suitably modified.

### Industrial Applicability

The present invention can be used in a light transmission/reception probe system and in a light transmission/reception probe.

### Reference Signs List

1 light transmission/reception probe system
10, 10A light transmission/reception probe
10a end portion (first end portion)
10a1 facet
10a2 facet
10a3 facet
10b end portion (second end portion)
10b1 inclined surface (optical element)
10c outer periphery
10d core
10e first cladding
10f second cladding (first enveloping layer)
11 leak enabling section
11a concave portion
11b particle
11c hole
12 transmitting section
13 enveloping layer (second enveloping layer)
14 scattering layer
14a scattering element
15A to 15D optical element
15a inclined surface (optical element)
15b conical surface (optical element)
15c lens (optical element)
15d convex curve (optical element)
20 delivery optical fiber
20a core
20b cladding
20c envelope
21 delivery optical fiber (second transmission optical fiber, second coupling section)
22 delivery optical fiber (first transmission optical fiber, first coupling section)
23 coupler
30 coupling section (first coupling section, second coupling section, integrated coupling section)
31 tapered section
32 linear section
100, 100A light output device
110 light source unit
111 light source unit
112 light source unit
200 control device
210 controller
211 irradiation control unit
212 input control unit
213 output control unit
214 detecting unit
220 input unit
230 output unit
241 main memory unit
242 auxiliary storage device
250 light receiving unit
A irradiation range
At end portion
Ax optical axis
Lc first light
Lr second light
X axial direction

## Claims

1. A light transmission/reception probe system comprising:
at least one light source;
a light transmission/reception probe including
a first end portion in an axial direction,
a second end portion on an opposite side of the first end portion in the axial direction,
a core,
a first cladding enclosing the core, and
a leak enabling section configured to enable leakage of first light from an outer periphery of the first cladding toward an outside in a radial direction, the first light being output from the light source, coupled with the first cladding in the first end portion, and transmitted through the first cladding;
a light receiving unit configured to receive second light, the second light arriving from an outside, being coupled with the core in the second end portion, being transmitted through the core, and being output from the core in the first end portion; and
a detecting unit configured to detect the second light received by the light receiving unit.

2. The light transmission/reception probe system according to claim 1, further comprising an integrated coupling section including
a first coupling section facing a position that is deviated from an axis center of the first end portion toward the outside in the radial direction, the first coupling section being configured to couple the light output from the at least one light source with the first cladding, and
a second coupling section facing a position closer to the axis center of the first end portion as compared to first coupling section, the second coupling section being configured to couple the light output from the core at the first end portion.

3. The light transmission/reception probe system according to claim 1 or 2, wherein the light transmission/reception probe includes a first enveloping layer configured to enclose the outer periphery of the first cladding in between the first end portion and the leak enabling section.

4. The light transmission/reception probe system according to claim 3, wherein the first enveloping layer includes a second cladding having a lower refractive index than a refractive index of the first cladding.

5. The light transmission/reception probe system according to any one of claims 1 to 4, wherein the light transmission/reception probe includes an envelope configured to enclose an outermost side of the light transmission/reception probe in the radial direction.

6. The light transmission/reception probe system according to claim 5, wherein the envelope is made of a resin material.

7. The light transmission/reception probe system according to claim 1 or 2, wherein the light transmission/reception probe includes a section in which outer periphery of the first cladding is exposed at a position deviated from the leak enabling section in the axial direction.

8. The light transmission/reception probe system according to any one of claims 1 to 7, wherein the light transmission/reception probe includes a second enveloping layer configured to
enclose the first cladding on the opposite side of the first end portion in the axial direction with respect to the leak enabling section, and
restrict direct coupling of the first light with the core in the leak enabling section.

9. The light transmission/reception probe system according to any one of claims 1 to 8, further comprising an optical element configured to change a direction of the light travelling toward an inside in the radial direction toward the core in the second end portion.

10. The light transmission/reception probe system according to any one of claims 1 to 9, wherein the leak enabling section includes a concave portion or a convex portion on the outer periphery of the first cladding.

11. The light transmission/reception probe system according to any one of claims 1 to 10, wherein the leak enabling section includes a particle or a hole inside the first cladding.

12. The light transmission/reception probe system according to any one of claims 1 to 11, wherein the leak enabling section includes a section in which a shape of the outer periphery goes on changing with advancement in the axial direction.

13. The light transmission/reception probe system according to any one of claims 1 to 12, wherein the leak enabling section includes a bent portion of the first cladding.

14. The light transmission/reception probe system according to any one of claims 1 to 13, wherein the leak enabling section includes a scattering layer arranged on an outside of the first cladding in the radial direction and configured to introduce light coming from the first cladding and cause scattering of the light toward the outside in the radial direction.

15. The light transmission/reception probe system according to claim 2, wherein the integrated coupling section is optically connected to the first end portion.

16. The light transmission/reception probe system according to claim 2 or 15, wherein the integrated coupling section includes a fiber bundle formed by bundling:
a first transmission optical fiber configured to transmit the first light, the first transmission optical fiber having an end portion serving as the first coupling section; and
a second transmission optical fiber configured to transmit the second light, the second transmission optical fiber having an end portion serving as the second coupling section.

17. The light transmission/reception probe system according to claim 16, wherein the fiber bundle is a tapered fiber bundle that goes on tapering toward the light transmission/reception probe.

18. The light transmission/reception probe system according to any one of claims 1 to 17, wherein
the at least one light source and the core in the first end portion are optically connected to each other, and
third light output from the light source and transmitted through the core is outputable from the second end portion.

19. The light transmission/reception probe system according to any one of claims 1 to 18, further comprising:
a plurality of light sources serving as the at least one light source; and
a control unit configured to switch among light sources for outputting light from among the plurality of light sources.

20. The light transmission/reception probe system according to claim 19, wherein the plurality of light sources include a plurality of light sources configured to output the first light.

21. The light transmission/reception probe system according to claim 19 or 20, wherein
the at least one light source and the core in the first end portion are optically connected to each other,
third light output from the light source and transmitted through the core is outputable from the second end portion, and
the plurality of light sources include light sources configured to output the first light and the third light.

22. The light transmission/reception probe system according to claim 19 to 21, further comprising an operation input unit configured to receive an operation input performed by a user, wherein
the control unit is configured to switch among light sources for outputting light from among the plurality of light sources in response to the operation input performed by the user using the operation input unit.

23. The light transmission/reception probe system according to any one of claims 1 to 22, wherein the light transmission/reception probe includes, as the core, a plurality of cores arranged in parallel.

24. A light transmission/reception probe comprising:
a first end portion in an axial direction;
a second end portion on an opposite side of the first end portion in the axial direction;
a core configured to extend in the axial direction;
a first cladding configured to enclose the core and extend in the axial direction; and
a leak enabling section disposed closer to the second end portion than the first end portion of the first cladding, and configured to allow leakage of first light from an outer periphery of the first cladding toward an outside in a radial direction, the first light being output from a light source, coupled with the first cladding in the first end portion, and transmitted through the first cladding.

25. The light transmission/reception probe according to claim 24, further comprising an integrated coupling section including
a first coupling section facing a position that is deviated from an axis center of the first end portion toward the outside in the radial direction, the first coupling section being configured to couple the light output from at least one light source with the first cladding, and
a second coupling section facing a position closer to the axis center of the first end portion as compared to the first coupling section, and configured to couple the light output from the core at the first end portion.

26. The light transmission/reception probe according to claim 24 or 25, further comprising a first enveloping layer configured to enclose an outer periphery of the first cladding in between the first end portion and the leak enabling section.

27. The light transmission/reception probe according to claim 26, wherein the first enveloping layer includes a second cladding having a lower refractive index than a refractive index of the first cladding.

28. The light transmission/reception probe according to any one of claims 24 to 27, further comprising an envelope configured to enclose an outermost side in the radial direction.

29. The light transmission/reception probe according to any one of claims 24 to 28, further comprising a second enveloping layer configured to
enclose the first cladding on the opposite side of the first end portion in the axial direction with respect to the leak enabling section, and
restrict direct coupling of the first light with the core in the leak enabling section.

30. The light transmission/reception probe according to any one of claims 24 to 29, further comprising, in the second end portion, an optical element configured to change a direction of the light travelling toward an inside in the radial direction toward the core.
